# EUROPEAN PATENT APPLICATION

(11) **EP 4 613 757 A1**
(43) Date of publication of application: **10.09.2025**
(21) Application number: 23886201.5
(22) Date of filing: 31.10.2023
(51) Int. Cl.: C07K 1/22, C07K 16/00

(54) **METHOD FOR REMOVING IMPURITIES THROUGH AFFINITY CHROMATOGRAPHY**

(30) Priority: 31.10.2022 KR 20220143067
(71) Applicant: Samsung Bioepis Co., Ltd., Yeonsu-gu Incheon 21987 (KR)
(72) Inventor: JUNG, Se Cheon, Incheon 21987 (KR); KIM, Song Young, Incheon 21987 (KR); SEO, Jeong Geol, Incheon 21987 (KR); OH, Eun Seo, Incheon 21987 (KR); KIM, Sang Wook, Incheon 21987 (KR); LEE, Tae Soo, Incheon 21987 (KR); PARK, Yeon Jin, Incheon 21987 (KR)
(74) Representative: Boult Wade Tennant LLP
(86) International application number: PCT/KR2023/017083
(87) International publication number: WO 2024/096505

(57) **Abstract**

The present disclosure relates to a composition for improving the removal of impurities in protein purification by affinity chromatography, the composition including a nonionic surfactant, and to a method of purifying a target protein by using the composition.

The composition or method according to an aspect, when used to purify a target protein, can effectively remove HCP while improving the yield of the target protein and thus may be applicable to manufacturing processes for recombinant protein pharmaceuticals such as antibody therapeutics.

## Description

### Technical Field

The present disclosure relates to a composition for improving the removal of impurities in protein purification by affinity chromatography, the composition including a nonionic surfactant, and to a method of purifying a target protein by using the composition.

### Background Art

Host cell protein (HCP) refers to all process-related impurities generated during the metabolic processes of host cells used for biopharmaceutical production. HCP, even in trace amounts, is known to have the potential to cause abnormal reactions such as immune responses or the formation of anti-drug antibodies. Therefore, HCP removal technology in the purification process is of significant importance.

As an affinity chromatography method, Protein A chromatography uses, as a ligand, Protein A that has specific binding affinity for the Fc fragment of an antibody. Due to this characteristic, Protein A chromatography is primarily employed as the first step in the antibody purification process, enabling the purification of antibodies to high purity from harvest cell culture fluid (HCCF) containing numerous impurities.

When HCCF (harvest cell culture fluid) is loaded onto a Protein A resin, the Fc fragment of the antibody binds to the Protein A ligand, while HCP forms nonspecific bonds with either the antibody or the Protein A resin. Due to this nonspecific binding, when the antibody is recovered (elution step) from the Protein A ligand, HCP nonspecifically bound to the antibody may be recovered as well.

Therefore, before antibody recovery, an additive capable of removing nonspecifically bound HCP can be applied (washing step) to remove these HCPs. However, depending on the type of additive, not only HCP but also the antibody bound to the Protein A ligand may be removed, resulting in reduced process yield.

Accordingly, there is a demand for the development of a technology capable of effectively reducing HCP in purified products from Protein A chromatography processes while maintaining purification process yield.

### Disclosure of Invention

### Technical Problem

An aspect provides a method of purifying a target protein, the method including: A) mixing a sample including a target protein with a composition including a nonionic surfactant; and B) removing impurities from the mixture by using affinity chromatography.

Another aspect provides a method of improving the removal of impurities in protein purification by affinity chromatography, the method including: A) mixing a sample including a target protein with a composition including a nonionic surfactant; and B) removing impurities from the mixture by using affinity chromatography.

Another aspect provides a composition for improving the removal of impurities in protein purification by affinity chromatography, the composition including a nonionic surfactant.

### Solution to Problem

An aspect provides a method of purifying a target protein, the method including: A) mixing a sample including a target protein with a composition including a nonionic surfactant; and B) removing impurities from the mixture by using affinity chromatography. The same details described below equally apply to the above method.

The method may be a method for removing impurities in a target protein purification process, and specifically, to a method for improving the removal of impurities.

The term "nonionic surfactant" in the present specification refers to a surfactant that dissolves in water without ionization and, unlike other types of surfactants, does not exhibit ionic properties. Because of this nonionic characteristic, the nonionic surfactant has good compatibility with other substances, offers a wide range of applications, and is therefore broadly employed in various fields.

The nonionic surfactant may include one or more selected from the group consisting of Triton X-100 (Triton X-100), Tergitol 15-S (Tergitol 15-S) series, Tergitol NP (Tergitol NP) series, Tergitol MIN-FOAM (Tergitol MIN-FOAM) series, and EcoSurf series, and may specifically be a Tergitol 15-S (Tergitol 15-S) series surfactant.

The term "Tergitol" in the present specification is a trade name for compounds commercially available from The Dow Chemical Company.

The nonionic surfactant may be a compound in the form of a secondary alcohol ethoxylate. Specifically, the nonionic surfactant may include (a) an aliphatic chain containing a specified number of carbons within the chain and (b) a specified number of ethoxylated units. The nonionic surfactant may be represented by Formula 1 below. 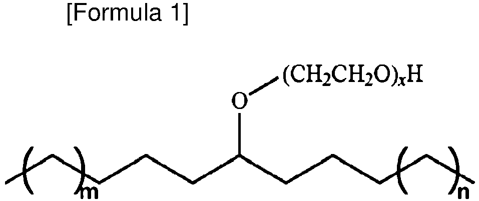 (m and n are each an integer greater than 0)

The nonionic surfactant may include Tergitol from the Tergitol 15-S series. Tergitol 15-S is a secondary alcohol ethoxylate compound with the chemical formula name of Alcohols, C12-14-secondary, ethoxylated, having an alkyl carbon chain length of 11 to 15 and containing 3 to 40 ethoxylate units. Tergitol 15-S may be represented by CAS No. 84133-50-6 and may be represented by Formula 2. 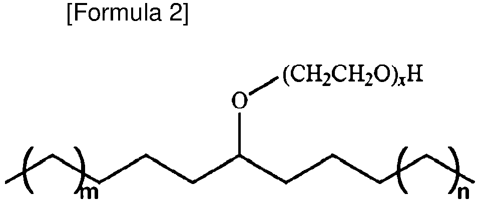 (m and n are each an integer greater than 0, the sum of m and n is an integer of 2 to 6, and x is an integer of 3 to 40)

Tergitol in the 15-S series may be denoted as "Tergitol 15-S-x" where x indicates the number of ethoxylate units. In Formula 2, x may be an integer of 3 to 40, specifically 3 to 40, 3 to 35, 3 to 30, 3 to 25, 3 to 20, 3 to 15, 3 to 12, 3 to 10, 3 to 9, 5 to 40, 5 to 35, 5 to 30, 5 to 25, 5 to 20, 5 to 15, 5 to 12, 5 to 10, 5 to 9, 7 to 40, 7 to 35, 7 to 30, 7 to 25, 7 to 20, 7 to 15, 7 to 12, 7 to 10, or 7 to 9.

Accordingly, for example, Tergitol 15-S-9 refers to a compound that includes 9 ethoxylate units and may be represented by Formula 3 below. 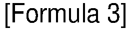 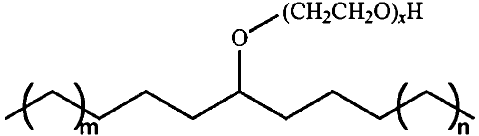 (where m and n are each an integer greater than 0, the sum of m and n is an integer of 2 to 6, and x is 9).

The 15-S series of Tergitol (Tergitol 15-S series) may include one or more selected from the group consisting of Tergitol 15-S-3, Tergitol 15-S-5, Tergitol 15-S-7, Tergitol 15-S-9, Tergitol 15-S-12, Tergitol 15-S-15, Tergitol 15-S-30, and Tergitol 15-S-40, and may specifically include Tergitol 15-S-9.

The NP series of Tergitol (Tergitol NP series) is a nonionic surfactant of the nonylphenol ethoxylate group, and may include one or more selected from the group consisting of Tergitol NP-4, Tergitol NP-6, Tergitol NP-7, Tergitol NP-8, Tergitol NP-9, Tergitol NP-9.5, Tergitol NP-10, Tergitol NP-11, Tergitol NP-12, Tergitol NP-13, Tergitol NP-15, Tergitol NP-30, Tergitol NP-40, Tergitol NP-50, Tergitol NP-55, and Tergitol NP-70.

The nonionic surfactant may include one or more selected from among alcohol ethoxylates and alkylphenol ethoxylates. The nonionic surfactant may include one or more selected from the group consisting of primary alcohol ethoxylates, secondary alcohol ethoxylates, nonylphenol ethoxylates, and octylphenol ethoxylates, and may specifically include a secondary alcohol ethoxylate.

The nonionic surfactant may include a compound represented by Formula 1, may specifically include a compound represented by Formula 2, and may more specifically include a compound represented by Formula 3.

The nonionic surfactant (specifically, Tergitol) may be included at a concentration of 0.003 %(w/w) to 2.0 %(w/w) with respect to the total weight of the mixture. Specifically, the concentration of the nonionic surfactant (specifically, Tergitol) in the mixture may be 0.003 %(w/w) to 2.0 %(w/w), 0.003 %(w/w) to 1.8 %(w/w), 0.003 %(w/w) to 1.6 %(w/w), 0.003 %(w/w) to 1.4 %(w/w), 0.003 %(w/w) to 1.2 %(w/w), 0.003 %(w/w) to 1.0 %(w/w), 0.003 %(w/w) to 0.9 %(w/w), 0.003 %(w/w) to 0.8 %(w/w), 0.003 %(w/w) to 0.7 %(w/w), 0.003 %(w/w) to 0.6 %(w/w), 0.003 %(w/w) to 0.5 %(w/w), 0.003 %(w/w) to 0.4 %(w/w), 0.003 %(w/w) to 0.3 %(w/w), 0.003 %(w/w) to 0.2 %(w/w), 0.003 %(w/w) to 0.1 %(w/w), 0.003 %(w/w) to 0.08 %(w/w), 0.003 %(w/w) to 0.06 %(w/w), 0.003 %(w/w) to 0.05 %(w/w), 0.003 %(w/w) to 0.04 %(w/w), 0.003 %(w/w) to 0.03 %(w/w), 0.003 %(w/w) to 0.02 %(w/w), 0.003 %(w/w) to 0.01 %(w/w), 0.003 %(w/w) to 0.008 %(w/w), 0.003 %(w/w) to 0.006 %(w/w), 0.003 %(w/w) to 0.005 %(w/w), 0.004 %(w/w) to 2.0 %(w/w), 0.004 %(w/w) to 1.8 %(w/w), 0.004 %(w/w) to 1.6 %(w/w), 0.004 %(w/w) to 1.4 %(w/w), 0.004 %(w/w) to 1.2 %(w/w), 0.004 %(w/w) to 1.0 %(w/w), 0.004 %(w/w) to 0.9 %(w/w), 0.004 %(w/w) to 0.8 %(w/w), 0.004 %(w/w) to 0.7 %(w/w), 0.004 %(w/w) to 0.6 %(w/w), 0.004 %(w/w) to 0.5 %(w/w), 0.004 %(w/w) to 0.4 %(w/w), 0.004 %(w/w) to 0.3 %(w/w), 0.004 %(w/w) to 0.2 %(w/w), 0.004 %(w/w) to 0.1 %(w/w), 0.004 %(w/w) to 0.08 %(w/w), 0.004 %(w/w) to 0.06 %(w/w), 0.004 %(w/w) to 0.05 %(w/w), 0.004 %(w/w) to 0.04 %(w/w), 0.004 %(w/w) to 0.03 %(w/w), 0.004 %(w/w) to 0.02 %(w/w), 0.004 %(w/w) to 0.01 %(w/w), 0.004 %(w/w) to 0.008 %(w/w), 0.004 %(w/w) to 0.006 %(w/w), 0.004 %(w/w) to 0.005 %(w/w), 0.005 %(w/w) to 2.0 %(w/w), 0.005 %(w/w) to 1.8 %(w/w), 0.005 %(w/w) to 1.6 %(w/w), 0.005 %(w/w) to 1.4 %(w/w), 0.005 %(w/w) to 1.2 %(w/w), 0.005 %(w/w) to 1.0 %(w/w), 0.005 %(w/w) to 0.9 %(w/w), 0.005 %(w/w) to 0.8 %(w/w), 0.005 %(w/w) to 0.7 %(w/w), 0.005 %(w/w) to 0.6 %(w/w), 0.005 %(w/w) to 0.5 %(w/w), 0.005 %(w/w) to 0.4 %(w/w), 0.005 %(w/w) to 0.3 %(w/w), 0.005 %(w/w) to 0.2 %(w/w), 0.005 %(w/w) to 0.1 %(w/w), 0.005 %(w/w) to 0.08 %(w/w), 0.005 %(w/w) to 0.06 %(w/w), 0.005 %(w/w) to 0.05 %(w/w), 0.005 %(w/w) to 0.04 %(w/w), 0.005 %(w/w) to 0.03 %(w/w), 0.005 %(w/w) to 0.02 %(w/w), 0.005 %(w/w) to 0.01 %(w/w), 0.005 %(w/w) to 0.008 %(w/w), 0.005 %(w/w) to 0.006 %(w/w), 0.006 %(w/w) to 2.0 %(w/w), 0.006 %(w/w) to 1.8 %(w/w), 0.006 %(w/w) to 1.6 %(w/w), 0.006 %(w/w) to 1.4 %(w/w), 0.006 %(w/w) to 1.2 %(w/w), 0.006 %(w/w) to 1.0 %(w/w), 0.006 %(w/w) to 0.9 %(w/w), 0.006 %(w/w) to 0.8 %(w/w), 0.006 %(w/w) to 0.7 %(w/w), 0.006 %(w/w) to 0.6 %(w/w), 0.006 %(w/w) to 0.5 %(w/w), 0.006 %(w/w) to 0.4 %(w/w), 0.006 %(w/w) to 0.3 %(w/w), 0.006 %(w/w) to 0.2 %(w/w), 0.006 %(w/w) to 0.1 %(w/w), 0.006 %(w/w) to 0.08 %(w/w), 0.006 %(w/w) to 0.06 %(w/w), 0.006 %(w/w) to 0.05 %(w/w), 0.006 %(w/w) to 0.04 %(w/w), 0.006 %(w/w) to 0.03 %(w/w), 0.006 %(w/w) to 0.02 %(w/w), 0.006 %(w/w) to 0.01 %(w/w), 0.006 %(w/w) to 0.008 %(w/w), 0.01 %(w/w) to 2.0 %(w/w), 0.01 %(w/w) to 1.8 %(w/w), 0.01 %(w/w) to 1.6 %(w/w), 0.01 %(w/w) to 1.4 %(w/w), 0.01 %(w/w) to 1.2 %(w/w), 0.01 %(w/w) to 1.0 %(w/w), 0.01 %(w/w) to 0.9 %(w/w), 0.01 %(w/w) to 0.8 %(w/w), 0.01 %(w/w) to 0.7 %(w/w), 0.01 %(w/w) to 0.6 %(w/w), 0.01 %(w/w) to 0.5 %(w/w), 0.01 %(w/w) to 0.4 %(w/w), 0.01 %(w/w) to 0.3 %(w/w), 0.01 %(w/w) to 0.2 %(w/w), 0.01 %(w/w) to 0.1 %(w/w), 0.01 %(w/w) to 0.08 %(w/w), 0.01 %(w/w) to 0.06 %(w/w), 0.01 %(w/w) to 0.05 %(w/w), 0.01 %(w/w) to 0.04 %(w/w), 0.01 %(w/w) to 0.03 %(w/w), 0.01 %(w/w) to 0.02 %(w/w), 0.03 %(w/w) to 2.0 %(w/w), 0.03 %(w/w) to 1.8 %(w/w), 0.03 %(w/w) to 1.6 %(w/w), 0.03 %(w/w) to 1.4 %(w/w), 0.03 %(w/w) to 1.2 %(w/w), 0.03 %(w/w) to 1.0 %(w/w), 0.03 %(w/w) to 0.9 %(w/w), 0.03 %(w/w) to 0.8 %(w/w), 0.03 %(w/w) to 0.7 %(w/w), 0.03 %(w/w) to 0.6 %(w/w), 0.03 %(w/w) to 0.5 %(w/w), 0.03 %(w/w) to 0.4 %(w/w), 0.03 %(w/w) to 0.3 %(w/w), 0.03 %(w/w) to 0.2 %(w/w), 0.03 %(w/w) to 0.1 %(w/w), 0.03 %(w/w) to 0.08 %(w/w), 0.03 %(w/w) to 0.06 %(w/w), 0.03 %(w/w) to 0.05 %(w/w), 0.03 %(w/w) to 0.04 %(w/w), 0.04 %(w/w) to 2.0 %(w/w), 0.04 %(w/w) to 1.8 %(w/w), 0.04 %(w/w) to 1.6 %(w/w), 0.04 %(w/w) to 1.4 %(w/w), 0.04 %(w/w) to 1.2 %(w/w), 0.04 %(w/w) to 1.0 %(w/w), 0.04 %(w/w) to 0.9 %(w/w), 0.04 %(w/w) to 0.8 %(w/w), 0.04 %(w/w) to 0.7 %(w/w), 0.04 %(w/w) to 0.6 %(w/w), 0.04 %(w/w) to 0.5 %(w/w), 0.04 %(w/w) to 0.4 %(w/w), 0.04 %(w/w) to 0.3 %(w/w), 0.04 %(w/w) to 0.2 %(w/w), 0.04 %(w/w) to 0.1 %(w/w), 0.04 %(w/w) to 0.08 %(w/w), 0.04 %(w/w) to 0.06 %(w/w), 0.04 %(w/w) to 0.05 %(w/w), 0.05 %(w/w) to 2.0 %(w/w), 0.05 %(w/w) to 1.8 %(w/w), 0.05 %(w/w) to 1.6 %(w/w), 0.05 %(w/w) to 1.4 %(w/w), 0.05 %(w/w) to 1.2 %(w/w), 0.05 %(w/w) to 1.0 %(w/w), 0.05 %(w/w) to 0.9 %(w/w), 0.05 %(w/w) to 0.8 %(w/w), 0.05 %(w/w) to 0.7 %(w/w), 0.05 %(w/w) to 0.6 %(w/w), 0.05 %(w/w) to 0.5 %(w/w), 0.05 %(w/w) to 0.4 %(w/w), 0.05 %(w/w) to 0.3 %(w/w), 0.05 %(w/w) to 0.2 %(w/w), 0.05 %(w/w) to 0.1 %(w/w), 0.05 %(w/w) to 0.08 %(w/w), 0.05 %(w/w) to 0.06 %(w/w), 0.1 %(w/w) to 2.0 %(w/w), 0.1 %(w/w) to 1.8 %(w/w), 0.1 %(w/w) to 1.6 %(w/w), 0.1 %(w/w) to 1.4 %(w/w), 0.1 %(w/w) to 1.2 %(w/w), 0.1 %(w/w) to 1.0 %(w/w), 0.1 %(w/w) to 0.9 %(w/w), 0.1 %(w/w) to 0.8 %(w/w), 0.1 %(w/w) to 0.7 %(w/w), 0.1 %(w/w) to 0.6 %(w/w), 0.1 %(w/w) to 0.5 %(w/w), 0.3 %(w/w) to 2.0 %(w/w), 0.3 %(w/w) to 1.8 %(w/w), 0.3 %(w/w) to 1.6 %(w/w), 0.3 %(w/w) to 1.4 %(w/w), 0.3 %(w/w) to 1.2 %(w/w), 0.3 %(w/w) to 1.0 %(w/w), 0.3 %(w/w) to 0.9 %(w/w), 0.3 %(w/w) to 0.8 %(w/w), 0.3 %(w/w) to 0.7 %(w/w), 0.3 %(w/w) to 0.6 %(w/w), 0.3 %(w/w) to 0.5 %(w/w), 0.4 %(w/w) to 2.0 %(w/w), 0.4 %(w/w) to 1.8 %(w/w), 0.4 %(w/w) to 1.6 %(w/w), 0.4 %(w/w) to 1.4 %(w/w), 0.4 %(w/w) to 1.2 %(w/w), 0.4 %(w/w) to 1.0 %(w/w), 0.4 %(w/w) to 0.9 %(w/w), 0.4 %(w/w) to 0.8 %(w/w), 0.4 %(w/w) to 0.7 %(w/w), 0.4 %(w/w) to 0.6 %(w/w), 0.4 %(w/w) to 0.5 %(w/w), 0.5 %(w/w) to 2.0 %(w/w), 0.5 %(w/w) to 1.8 %(w/w), 0.5 %(w/w) to 1.6 %(w/w), 0.5 %(w/w) to 1.4 %(w/w), 0.5 %(w/w) to 1.2 %(w/w), 0.5 %(w/w) to 1.0 %(w/w), 0.5 %(w/w) to 0.9 %(w/w), 0.5 %(w/w) to 0.8 %(w/w), 0.5 %(w/w) to 0.7 %(w/w), or 0.5 %(w/w) to 0.6 %(w/w).

The nonionic surfactant may inhibit or suppress the nonspecific binding of impurities in the sample, and specifically, may inhibit or suppress one or more of 1) the nonspecific binding between an impurity in the sample and the target protein, and 2) the nonspecific binding between an impurity in the sample and the affinity ligand of the affinity chromatography. By inhibiting nonspecific binding of impurities, the nonionic surfactant may effectively remove impurities during the target protein purification process that employs affinity chromatography.

In the present specification, the term "target protein" refers to any protein that a person skilled in the art intends to express or obtain, including any protein capable of being expressed in a host cell or in a desired organism. In the present specification, the target protein may be used interchangeably with "target protein" or "protein of interest".

The target protein may be a recombinant protein and for example, may include those used as antibody therapeutics, anti-cancer immunotherapeutics, cancer treatment vaccines, infectious disease vaccines, protein replacement therapeutics, allergy therapeutics, or immunotherapeutics for immune-related diseases.

The target protein may be selected from the group consisting of: antibodies or antigen-binding fragments thereof, immunoadhesins, Transforming Growth Factor (TGF)-beta superfamily signaling molecules, blood coagulation factors, anti-tumor necrosis factor receptor (TNFR) antibodies, anti-human epidermal growth factor receptor 2 (HER2) antibodies, and TNFR:Fc.

In the present specification, the term "antibody" is used interchangeably with the term "immunoglobulin (Ig)". A complete antibody has two full-length light chains and two full-length heavy chains, and each light chain is bound to a heavy chain via a disulfide bond (SS-bond). The antibody may be, for example, IgA, IgD, IgE, IgG, or IgM. The antibody may be a monoclonal antibody or a polyclonal antibody. The antibody may be an animal-derived antibody, a mouse-human chimeric antibody, a humanized antibody, or a human antibody. As used herein, the term "antigen-binding fragment" refers to a fragment of the complete immunoglobulin structure, specifically referring to a portion of the polypeptide that contains the antigen-binding region. For example, the antigen-binding fragment may be scFv, (scFv)₂, Fv, Fab, Fab', Fv F(ab')₂, or a combination thereof.

The immunoadhesin is an antibody-like protein, and refers to a fusion protein of an immunoglobulin Fc region and a functional domain of a binding protein (e.g., a receptor, ligand, or cell-adhesion molecule).

The TGF-beta superfamily signaling molecule may be, for example, TGF-beta 1, TGF-beta 2, or TGF-beta 3. The blood coagulation factor refers to a factor involved in blood coagulation. The blood coagulation factor may be, for example, fibrinogen, prothrombin, tissue thromboplastin, Factor V, Factor VII, Factor VIII, Factor IX, Factor X, Factor XI, Factor XII, Factor XIII, von Willebrand Factor, prekallikrein, high-molecular-weight kininogen, fibronectin, antithrombin III, heparin cofactor II, Protein C, Protein S, Protein Z, Protein Z-dependent protease inhibitor, plasminogen, alpha 2-antiplasmin, tissue plasminogen activator, urokinase, plasminogen activator inhibitor-1, plasminogen activator inhibitor-2, or cancer procoagulant.

The anti-TNFR antibody may be an antibody that specifically binds to TNFR or an antigen-binding fragment thereof. In addition, the anti-HER2 antibody may be an antibody that specifically binds to HER2 or an antigen-binding fragment thereof, and TNFR:Fc refers to a fusion protein of TNFR and an Fc region.

The target protein may be selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, urelumab, adornase alfa, REBIF, becaplermin, alteplase, laronidase, alefacept, aflibercept, raxibacumab, darbepoetin alfa, becaplermin concentrate, interferon beta-1b, Botulinum toxin type A, rasburicase, asparaginase, epoetin alfa, etanercept, agalsidase beta, interferon alfacon-1, interferon alfa-2a, anakinra, Botulinum toxin type B, pegfilgrastim, oprelvekin, filgrastim, denileukin diftitox, peginterferon alfa-2a, aldesleukin, dornase alfa, interferon beta-1a, becaplermin, reteplase, interferon alfa-2, tenecteplase, drotrecogin alfa, rilonacept, romiplostim, methoxypolyethylene glycol-epoetin beta, C1 esterase inhibitor, idursulfase, alglucosidase alfa, abatacept, galsulfase, palifermin, and interferon gamma-1b.

The target protein may be a protein (Fc-containing protein) that contains an Fc fragment, such as an immunoglobulin or an Fc-fusion protein. The term "Fc fragment," as used herein, refers to the crystallizable tail region of an antibody that interacts with cell surface receptors called Fc receptors, and may be understood as the amino acid residues of an immunoglobulin molecule capable of interacting with Protein A. The term "Fc fragment" as used herein may be used interchangeably with "Fc region", "Fc domain", or "Fc".

The sample containing the target protein may be a cell culture fluid, and specifically may be a culture fluid obtained from a host cell or an organism that expresses/produces the target protein.

The term "cell culture fluid" as used herein means a harvest cell culture fluid (HCCF), a cell culture supernatant, or a pretreated cell culture fluid/culture supernatant. The cell culture fluid may be directly obtained from the host cell or organism that expresses/produces the target protein. The cell culture fluid may have been partially purified or refined by centrifugation and/or filtration, for example, microfiltration, diafiltration, ultrafiltration, or depth filtration.

The term "pretreated" as used herein refers to a cell culture fluid that is prepared, for example, for the chromatography step used in the method of the present disclosure by performing one or more adjustments-such as buffer exchange, dilution, addition of salt, detergent, chaotropic agent, or organic compound, pH titration, or filtration-for the purpose of achieving the desired chromatographic performance and stabilizing the target protein, through adjustment of pH and/or conductivity range and/or buffering capacity. Because the target protein expressed by mammalian cells is generally secreted into the cell culture fluid during the culture process, product harvesting at the end of the culture process is accomplished by separating the cell culture fluid from the cells. The cell separation method must be carried out gently to minimize cell disruption in order to avoid increased cell debris, and the release of protease and other molecules that may affect the quality of the target protein product. Generally, harvested material from mammalian cell cultures undergoes centrifugation followed by filtration. Expanded bed adsorption chromatography is an alternative method intended to avoid centrifugation/filtration steps. Additional treatments of the sample prior to purification through a chromatography step may include concentrating and/or diafiltering the cell culture fluid to achieve a specific target protein concentration, pH range, conductivity, or buffer species concentration.

The host cell or organism that produces the target protein may be transformed with a gene encoding the target protein, and may be any suitable host cell or organism capable of expressing the target protein. The host cell may be eukaryotic or prokaryotic, and may include, without limitation, bacterial cells, yeast cells, insect cells, and mammalian cells, with mammalian cells being preferred. The host cell may be eukaryotic or prokaryotic, including, without being limited to, bacterial cells, yeast cells, insect cells, and mammalian cells, with mammalian cells being preferred. Non-limiting examples of suitable mammalian cells may include antibody-producing hybridoma cells, as well as expression hosts such as COS7 (monkey kidney) cells, NSO cells, SP2/0 cells, Chinese hamster ovary (CHO) cells, W138 cells, baby hamster kidney (BHK) cells, MDCK cells, myeloma cell lines, HeLa cells, HuT 78 cells, or HEK293 cells.

Specifically, step A) may include adding and mixing a composition containing a nonionic surfactant or the nonionic surfactant itself into a sample containing the target protein, specifically, a cell culture fluid. This may further include adding a fixed concentration of the nonionic surfactant to the sample, followed by filtration and/or incubation. In addition, in order to effectively carry out affinity chromatography for the purpose of obtaining the target protein at high efficiency or effectively removing impurities, the method may further include a step of treating the sample with additional components or introducing additional processes before or after adding the composition containing a nonionic surfactant or the nonionic surfactant.

In the method, step B) is a step of removing impurities from the mixture by using affinity chromatography and, specifically, of purifying the target protein by removing impurities.

As used herein, the term "affinity chromatography" or "affinity purification" refers to a separation method based on the specific binding interaction between a ligand, which is immobilized or coupled on a solid support, and its binding partner. When a complex mixture passes through the column, molecules that have specific binding affinity for the affinity ligand become bound. After other sample components are removed by washing, the bound molecules are eluted from the support, resulting in their purification from the original mixture. Each specific affinity system requires its own set of conditions as known to those skilled in the art.

The affinity ligand may be selected from among metals (e.g., Cd⁺², Co⁺², Cu⁺², Ga+³, Fe⁺³, Ni⁺², and Zn⁺²), dyes (e.g., Cibacron Blue and its variants), glutathione, subtilisin, Protein A, Protein G, Protein A/G, Protein L, boronate, avidin, streptavidin, biotin, anti-c-Myc, anti-HA, nucleotides, coenzymes, antibodies, heparin, antigens (for antibodies with known specificity), and other known affinity ligands.

According to one example, the affinity chromatography may be selected from among Protein A chromatography, Capto Blue (High Sub) chromatography, Protein G chromatography, Protein A/G chromatography, Protein L chromatography, Lambda Fab Select chromatography, Kappa Select chromatography, Ig Select chromatography, Blue Sepharose chromatography, Capto Heparin chromatography, VII Select chromatography, VIII Select chromatography, XSelect chromatography, and Capto L chromatography, and may specifically be Protein A chromatography.

The term "Protein A chromatography" as used herein refers to a technique or method for separating or purifying substances and/or particles using Protein A. Protein A is a 40-60 kD cell wall protein originally found in Staphylococcus aureus and may include Protein A recovered from its natural source; Protein A produced by synthetic or biosynthetic methods (for example, by peptide synthesis or recombinant technology); and variants thereof that retain the ability to bind to proteins having a CH2/CH3 region and/or an Fc region. Protein A is generally immobilized on a solid phase; the term "Protein A" therefore refers to an affinity chromatography resin or column containing a chromatographic solid support matrix to which Protein A is covalently bound. As one example, a chromatography column for use in Protein A chromatography may include Protein A immobilized on a polyvinylether solid phase (for example, an Eshmuno^{®} column (Merck, Darmstadt, Germany)), Protein A immobilized on a porous glass matrix (for example, a ProSep^{®} column (Merck, Darmstadt, Germany)), or Protein A immobilized on an agarose solid phase (for example, a MABSELECT^{™} SuRe^{™} column (GE Healthcare, Uppsala, Sweden)), without being limited thereto.

In the present specification, the term "impurity" refers to any substance different from the target protein that is to be purified, recovered, or obtained in the above method. Such impurities may include cell culture medium components, cell debris, host cell proteins (HCP), endotoxins, viruses, lipids, DNA, RNA, leachables from process materials, and aggregates or fragments thereof; product-related substances such as aggregates, charge variants, misfolded molecules, or fragments of the target protein to be purified may also be regarded as impurities. In the present specification, the term "impurity" may be used interchangeably with "contaminant".

In the method, the impurity contained in the sample including the target protein, which is to be removed by the present disclosure, may include host cell proteins.

In the present specification, the term "host cell protein (HCP)" refers to all process-related impurities generated during the metabolic processes of host cells that express/produce the target protein. In the present specification, the host cell protein may be used interchangeably with "host cell protein contaminant" or "host cell protein impurity".

In the method, step B) may include:
B-1) loading the mixture onto an affinity chromatography column; and
B-2) washing the column with a first wash buffer.

The affinity chromatography column may be a column equilibrated with an equilibration buffer, specifically a column equilibrated with the equilibration buffer before loading the mixture. Therefore, step B-1) may further include equilibrating the affinity chromatography column with the equilibration buffer prior to loading the mixture.

Furthermore, after the mixture is loaded, the affinity chromatography column may be equilibrated with the equilibration buffer. Therefore, step B-1) may further include equilibrating the affinity chromatography column with the equilibration buffer after loading the mixture.

The equilibration buffer may include one or more salts selected from the group consisting of sodium phosphate, sodium chloride, Tris, Tris-hydrochloride (Tris-HCl), MES (2-(N-morpholino)ethanesulfonic acid), MOPS (3-morpholinopropane-1-sulfonic acid), PIPES, potassium phosphate, potassium chloride, and HEPES (2-[4-(2-hydroxyethyl)piperazin-1-yl]ethanesulfonic acid), without being limited thereto.

In an embodiment, the equilibration buffer may have a pH of 7 to 8, may contain sodium phosphate at a concentration of 50 mM to 100 mM, and may contain sodium chloride at a concentration of 70 mM to 130 mM.

The equilibration buffer may have a pH of 7 to 8, 7 to 7.8, 7 to 7.6, 7 to 7.4, 7 to 7.3, 7.2 to 8, 7.2 to 7.8, 7.2 to 7.6, 7.2 to 7.4, 7.2 to 7.3, 7.3 to 8, 7.3 to 7.8, 7.3 to 7.6, or 7.3 to 7.4.

The equilibration buffer may contain sodium phosphate at a concentration of 50 mM to 100 mM, 50 mM to 90 mM, 50 mM to 80 mM, 50 mM to 75 mM, 60 mM to 100 mM, 60 mM to 90 mM, 60 mM to 80 mM, 60 mM to 75 mM, 70 mM to 100 mM, 70 mM to 90 mM, 70 mM to 80 mM, 70 mM to 75 mM, 75 mM to 100 mM, 75 mM to 90 mM, or 75 mM to 80 mM.

The equilibration buffer may contain sodium phosphate at a concentration of 70 mM to 130 mM, 70 mM to 120 mM, 70 mM to 110 mM, 70 mM to 105 mM, 70 mM to 100 mM, 80 mM to 130 mM, 80 mM to 120 mM, 80 mM to 110 mM, 80 mM to 105 mM, 80 mM to 100 mM, 90 mM to 130 mM, 90 mM to 120 mM, 90 mM to 110 mM, 90 mM to 105 mM, 90 mM to 100 mM, 95 mM to 130 mM, 95 mM to 120 mM, 95 mM to 110 mM, 95 mM to 105 mM, 95 mM to 100 mM, 100 mM to 130 mM, 100 mM to 120 mM, 100 mM to 110 mM, or 100 mM to 105 mM.

Step B-2) may be a step for removing impurities such as host cell proteins present in the column by washing the column with a first wash buffer. Step B-2) may be performed one or more times.

Such washing may refer to the application of a mobile phase that elutes impurities from the stationary phase without eluting the target product (the target protein).

The first wash buffer may be a buffer containing a suitable component or combination of components for removing only impurities (including host cell proteins in the column) while maintaining the binding between the target protein and the affinity ligand. Specifically, the buffer may include one or more selected from the group consisting of sodium phosphate, sodium chloride, Tris, Tris-HCl, MES (2-(N-morpholino)ethanesulfonic acid), MOPS (3-morpholinopropane-1-sulfonic acid), PIPES, potassium phosphate, potassium chloride, HEPES (2-[4-(2-hydroxyethyl)piperazin-1-yl]ethanesulfonic acid), Triton X-100, urea, Tween 80, lauryldimethylamine oxide (LDAO), calcium chloride (CaCl₂), arginine, EDTA (ethylene-diamine-tetraacetic acid), guanidine, isopropanol, and acceptable salts thereof, without being limited thereto.

In an embodiment, the first wash buffer may not include one or more selected from among guanidine, guanidine salts (for example, guanidine-HCl), and isopropanol, and may specifically exclude all of guanidine, guanidine salts, and isopropanol.

In an embodiment, the first wash buffer may contain Tris at a concentration of 20 mM to 80 mM and/or sodium chloride at a concentration of 0.5 M to 1.5 M, and have a pH of 6.5 to 8.

The first wash buffer may have a pH of 6.5 to 8, 6.5 to 7.8, 6.5 to 7.6, 6.5 to 7.4, 6.5 to 7.2, 6.5 to 7.0, 6.7 to 8, 6.7 to 7.8, 6.7 to 7.6, 6.7 to 7.4, 6.7 to 7.2, 6.7 to 7.0, 7.0 to 8, 7.0 to 7.8, 7.0 to 7.6, 7.0 to 7.4, or 7.0 to 7.2.

The first wash buffer may include Tris at a concentration of 20 mM to 80 mM, 20 mM to 70 mM, 20 mM to 60 mM, 20 mM to 55 mM, 20 mM to 50 mM, 30 mM to 80 mM, 30 mM to 70 mM, 30 mM to 60 mM, 30 mM to 55 mM, 30 mM to 50 mM, 40 mM to 80 mM, 40 mM to 70 mM, 40 mM to 60 mM, 40 mM to 55 mM, 40 mM to 50 mM, 45 mM to 80 mM, 45 mM to 70 mM, 45 mM to 60 mM, 45 mM to 55 mM, 45 mM to 50 mM, 50 mM to 80 mM, 50 mM to 70 mM, 50 mM to 60 mM, or 50 mM to 55 mM.

The first wash buffer may include sodium chloride at a concentration of 0.5 M to 1.5 M, 0.5 M to 1.3 M, 0.5 M to 1.1 M, 0.5 M to 1.0 M, 0.7 M to 1.5 M, 0.7 M to 1.3 M, 0.7 M to 1.1 M, 0.7 M to 1.0 M, 0.9 M to 1.5 M, 0.9 M to 1.3 M, 0.9 M to 1.1 M, 0.9 M to 1.0 M, 1.0 M to 1.5 M, 1.0 M to 1.3 M, or 1.0 M to 1.1 M.

Step B) may further include step B-3) washing the column with a second wash buffer.

Step B-3) may be a step of removing impurities-such as the first wash buffer and its residues-that remain in the column, by washing the column (already washed with the first wash buffer) with the second wash buffer. Step B-3) may be carried out one or more times.

The second wash buffer may be a buffer containing a suitable component or a combination of components for removing only impurities (including the first wash buffer and its residues) from the column while maintaining the binding between the target protein and the affinity ligand. Specifically, the buffer may include one or more selected from the group consisting of sodium phosphate, sodium chloride, Tris, Tris-HCl, MES (2-(N-morpholino)ethanesulfonic acid), MOPS (3-morpholinopropane-1-sulfonic acid), PIPES, potassium phosphate, potassium chloride, HEPES (2-[4-(2-hydroxyethyl)piperazin-1-yl]ethanesulfonic acid), and acceptable salts thereof, without being limited thereto.

In an embodiment, the second wash buffer may have a pH of 6.5 to 8 and contain Tris at a concentration of 20 mM to 80 mM.

The second wash buffer may have a pH of 6.5 to 8, 6.5 to 7.8, 6.5 to 7.6, 6.5 to 7.4, 6.5 to 7.2, 6.7 to 8, 6.7 to 7.8, 6.7 to 7.6, 6.7 to 7.4, 6.7 to 7.2, 7.0 to 8, 7.0 to 7.8, 7.0 to 7.6, 7.0 to 7.4, 7.0 to 7.2, 7.2 to 8, 7.2 to 7.8, 7.2 to 7.6, or 7.2 to 7.4.

The second wash buffer may contain Tris at a concentration of 20 mM to 80 mM, 20 mM to 70 mM, 20 mM to 60 mM, 20 mM to 55 mM, 20 mM to 50 mM, 30 mM to 80 mM, 30 mM to 70 mM, 30 mM to 60 mM, 30 mM to 55 mM, 30 mM to 50 mM, 40 mM to 80 mM, 40 mM to 70 mM, 40 mM to 60 mM, 40 mM to 55 mM, 40 mM to 50 mM, 45 mM to 80 mM, 45 mM to 70 mM, 45 mM to 60 mM, 45 mM to 55 mM, 45 mM to 50 mM, 50 mM to 80 mM, 50 mM to 70 mM, 50 mM to 60 mM, or 50 mM to 55 mM.

The method may further include step C) recovering the target protein from the column by using an elution buffer. Step C) may be a step for recovering the target protein present in the column, and specifically, may be a step for recovering the target protein or a purified product containing the target protein by using an elution buffer.

The elution buffer is a buffer used to elute proteins from the chromatography column and may be a buffer containing suitable components or a combination of components that reduce the binding between the target protein and the affinity ligand, thereby enabling effective recovery of the target protein. Specifically, the elution buffer may include one or more selected from the group consisting of citrate, acetate, phosphate, ammonium, and glycine, without being limited thereto.

In an embodiment, the elution buffer may have a pH of 2 to 5 and may contain glycine at a concentration of 100 mM to 500 mM.

The elution buffer may have a pH of 1 to 5, 1 to 4.5, 1 to 4, 1 to 3.5, 1.5 to 5, 1.5 to 4.5, 1.5 to 4, 1.5 to 3.5, 2 to 5, 2 to 4.5, 2 to 4, 2 to 3.5, 2.5 to 5, 2.5 to 4.5, 2.5 to 4, 2.5 to 3.5, 3 to 5, 3 to 4.5, 3 to 4, or 3 to 3.5.

The elution buffer may contain glycine at a concentration of 100 mM to 500 mM, 100 mM to 450 mM, 100 mM to 400 mM, 100 mM to 350 mM, 100 mM to 300 mM, 150 mM to 500 mM, 150 mM to 450 mM, 150 mM to 400 mM, 150 mM to 350 mM, 150 mM to 300 mM, 200 mM to 500 mM, 200 mM to 450 mM, 200 mM to 400 mM, 200 mM to 350 mM, 200 mM to 300 mM, 250 mM to 500 mM, 250 mM to 450 mM, 250 mM to 400 mM, 250 mM to 350 mM, 250 mM to 300 mM, 300 mM to 500 mM, 300 mM to 450 mM, 300 mM to 400 mM, or 300 mM to 350 mM.

In step C), the eluate may be subjected to further processing in a subsequent step, for example, isothermal treatment at a lower pH, or a neutralization process.

The eluate, recovered product, or purified product obtained through the method may contain impurities (specifically, host cell proteins) at 1400 ppm or less, 1300 ppm or less, 1200 ppm or less, 1100 ppm or less, 1000 ppm or less, 900 ppm or less, 800 ppm or less, 700 ppm or less, or 600 ppm or less.

The eluate, recovered product, or purified product obtained through the method may contain impurities (specifically, host cell proteins) at 500 ppm to 1300 ppm, 500 ppm to 1200 ppm, 500 ppm to 1100 ppm, 500 ppm to 1000 ppm, 500 ppm to 950 ppm, 500 ppm to 900 ppm, 500 ppm to 850 ppm, 500 ppm to 800 ppm, 500 ppm to 750 ppm, 500 ppm to 700 ppm, 500 ppm to 650 ppm, 500 ppm to 600 ppm, 550 ppm to 1300 ppm, 550 ppm to 1200 ppm, 550 ppm to 1100 ppm, 550 ppm to 1000 ppm, 550 ppm to 950 ppm, 550 ppm to 900 ppm, 550 ppm to 850 ppm, 550 ppm to 800 ppm, 550 ppm to 750 ppm, 550 ppm to 700 ppm, 550 ppm to 650 ppm, 550 ppm to 600 ppm, 600 ppm to 1200 ppm, 600 ppm to 1100 ppm, 600 ppm to 1000 ppm, 600 ppm to 950 ppm, 600 ppm to 900 ppm, 600 ppm to 850 ppm, 600 ppm to 800 ppm, 600 ppm to 750 ppm, 600 ppm to 700 ppm, 600 ppm to 650 ppm, 650 ppm to 1200 ppm, 650 ppm to 1100 ppm, 650 ppm to 1000 ppm, 650 ppm to 950 ppm, 650 ppm to 900 ppm, 650 ppm to 850 ppm, 650 ppm to 800 ppm, 650 ppm to 750 ppm, or 650 ppm to 700 ppm.

The eluate, recovered product, or purified product obtained through the method may exhibit a target protein yield of 95 % to 105 %, 95 % to 104 %, 95 % to 103 %, 95 % to 102 %, 95 % to 101 %, 95 % to 100 %, 97 % to 105 %, 97 % to 104 %, 97 % to 103 %, 97 % to 102 %, 97 % to 101 %, 97 % to 100 %, 99 % to 105 %, 99 % to 104 %, 99 % to 103 %, 99 % to 102 %, 99 % to 101 %, or 99 % to 100 %. The target protein yield may be calculated as the ratio of the amount of target protein contained in the eluate, recovered product, or purified product obtained after applying affinity chromatography, to the amount of target protein contained in the sample containing the target protein before applying the affinity chromatography.

The eluate, recovered product, or purified product obtained through the method may exhibit more effective impurity removal than the eluate, recovered product, or purified product obtained without using a composition containing a nonionic surfactant. Specifically, when the method of the present disclosure is used, it is possible to obtain an eluate, recovered product, or purified product that has a lower impurity content while maintaining or even increasing the target protein yield, compared to a control that does not use a composition containing a nonionic surfactant.

The eluate, recovered product, or purified product obtained through the method may have a reduced level of impurities (specifically, host cell proteins) compared to an eluate, recovered product, or purified product obtained through affinity chromatography without using a composition containing a nonionic surfactant.

The amount of impurities contained in the eluate, recovered product, or purified product obtained through the method may be decreased by about 5 % or more compared to the amount of impurities contained in an eluate, recovered product, or purified product obtained through affinity chromatography without using a composition containing a nonionic surfactant, and specifically, may be decreased by about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The eluate, recovered product, or purified product obtained through the method may exhibit an increased or maintained yield (amount) of the target protein compared to an eluate, recovered product, or purified product obtained by affinity chromatography without using a composition containing a nonionic surfactant, and may specifically be increased above a certain level or increased/decreased below a certain level.

The yield (amount) of the target protein contained in the eluate, recovered product, or purified product obtained through the method may be increased by about 5 % or more than the yield (amount) of the target protein contained in an eluate, recovered product, or purified product obtained by affinity chromatography without using a composition containing a nonionic surfactant. Specifically, the increase in the yield (amount) of the target protein may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The yield (amount) of the target protein contained in the eluate, recovered product, or purified product obtained through the method may be increased/decreased by about 10 % compared to the yield (amount) of the target protein contained in an eluate, recovered product, or purified product obtained by affinity chromatography without using a composition containing a nonionic surfactant, and specifically, may be increased/decreased by about 10 % or less, about 8 % or less, about 5 % or less, about 3 % or less, about 2 % or less, about 1 % or less, about 0.5 % or less, about 0.1 % or less, about 0.01 % or less, about 0.01 % to about 10 %, about 0.01 % to about 8 %, about 0.01 % to about 5 %, about 0.01 % to about 3 %, about 0.01 % to about 1 %, about 0.01 % to about 0.1 %, about 0.1 % to about 10 %, about 0.1 % to about 8 %, about 0.1 % to about 5 %, about 0.1 % to about 3 %, about 0.1 % to about 1 %, about 0.5 % to about 10 %, about 0.5 % to about 8 %, about 0.5 % to about 5 %, about 0.5 % to about 3 %, about 0.5 % to about 1 %, about 1 % to about 10 %, about 1 % to about 8 %, about 1 % to about 5 %, or about 1 % to about 3 %

The eluate, recovered product, or purified product obtained through the method may exhibit more effective impurity removal than an eluate, recovered product, or purified product obtained by affinity chromatography using a wash buffer that includes one or more selected from the group consisting of guanidine, guanidine salts, and isopropanol and/or without using a composition containing a nonionic surfactant. Specifically, using the method of the present disclosure may make it possible to obtain an eluate, recovered product, or purified product having a lower or comparable amount of impurities while achieving an increased yield of the target protein, compared to the control group that does not use a composition containing a nonionic surfactant and/or that uses a wash buffer including one or more selected from the group consisting of guanidine, guanidine salts, and isopropanol.

The eluate, recovered product, or purified product obtained through the method may have a decreased amount of impurities (specifically, host cell proteins) compared to the eluate, recovered product, or purified product obtained by performing affinity chromatography using a wash buffer containing one or more selected from the group consisting of guanidine, guanidine salts, and isopropanol and/or without using a composition containing a nonionic surfactant.

The amount of impurities in the eluate, recovered product, or purified product obtained through the method may be decreased by about 5 % or more compared to the amount of impurities in the eluate, recovered product, or purified product obtained by performing affinity chromatography using a wash buffer containing one or more selected from the group consisting of guanidine, guanidine salts, and isopropanol and/or without using a composition containing a nonionic surfactant. Specifically, the decrease in the amount of impurities may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %

Moreover, the eluate, recovered product, or purified product obtained through the method may exhibit an increased yield (amount) of the target protein compared to the eluate, recovered product, or purified product obtained by performing affinity chromatography using a wash buffer containing one or more selected from among guanidine, guanidine salts, and isopropanol and/or without using a composition containing a nonionic surfactant.

The yield (amount) of the target protein in the eluate, recovered product, or purified product obtained through the method may be increased by about 5 % or more than the yield (amount) of the target protein in the eluate, recovered product, or purified product obtained by performing affinity chromatography using a wash buffer containing one or more selected from among guanidine, guanidine salts, and isopropanol and/or without using a composition containing a nonionic surfactant. Specifically, the increase in the yield (amount) of the target protein may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

Another aspect provides a method for improving the removal of impurities in protein purification by affinity chromatography, the method including: A) mixing a sample including a target protein with a composition including a nonionic surfactant; and B) removing impurities from the mixture by using affinity chromatography. The same details as described above equally apply to the above method.

The method may be a method for purifying a target protein by improving the removal of impurities in protein purification.

Another aspect provides a method for producing a purified target protein by affinity chromatography, the method including: A) mixing a sample including a target protein with a composition including a nonionic surfactant; and B) removing impurities from the mixture by affinity chromatography. The same details as described above equally apply to the above method.

The method may be a method of purifying a target protein by improving the removal of impurities in protein purification.

Another aspect provides a composition for improving the removal of impurities in protein purification by affinity chromatography, the composition including a nonionic surfactant. The same details as described above equally apply to the above composition.

The composition may be a composition for purifying a target protein and/or producing a purified target protein by improving impurity removal in protein purification.

The composition may be used prior to applying a sample containing the target protein to affinity chromatography.

The composition may be mixed with the sample containing the target protein, such that the composition or the nonionic surfactant contained therein is included at a concentration of 0.003 %(w/w) to 2.0 %(w/w) based on the total weight of the mixture. Specifically, the concentration of the nonionic surfactant (specifically, Turgitol) in the mixture may be 0.003 %(w/w) to 2.0 %(w/w), 0.003 %(w/w) to 1.8 %(w/w), 0.003 %(w/w) to 1.6 %(w/w), 0.003 %(w/w) to 1.4 %(w/w), 0.003 %(w/w) to 1.2 %(w/w), 0.003 %(w/w) to 1.0 %(w/w), 0.003 %(w/w) to 0.9 %(w/w), 0.003 %(w/w) to 0.8 %(w/w), 0.003 %(w/w) to 0.7 %(w/w), 0.003 %(w/w) to 0.6 %(w/w), 0.003 %(w/w) to 0.5 %(w/w), 0.003 %(w/w) to 0.4 %(w/w), 0.003 %(w/w) to 0.3 %(w/w), 0.003 %(w/w) to 0.2 %(w/w), 0.003 %(w/w) to 0.1 %(w/w), 0.003 %(w/w) to 0.08 %(w/w), 0.003 %(w/w) to 0.06 %(w/w), 0.003 %(w/w) to 0.05 %(w/w), 0.003 %(w/w) to 0.04 %(w/w), 0.003 %(w/w) to 0.03 %(w/w), 0.003 %(w/w) to 0.02 %(w/w), 0.003 %(w/w) to 0.01 %(w/w), 0.003 %(w/w) to 0.008 %(w/w), 0.003 %(w/w) to 0.006 %(w/w), 0.003 %(w/w) to 0.005 %(w/w), 0.004 %(w/w) to 2.0 %(w/w), 0.004 %(w/w) to 1.8 %(w/w), 0.004 %(w/w) to 1.6 %(w/w), 0.004 %(w/w) to 1.4 %(w/w), 0.004 %(w/w) to 1.2 %(w/w), 0.004 %(w/w) to 1.0 %(w/w), 0.004 %(w/w) to 0.9 %(w/w), 0.004 %(w/w) to 0.8 %(w/w), 0.004 %(w/w) to 0.7 %(w/w), 0.004 %(w/w) to 0.6 %(w/w), 0.004 %(w/w) to 0.5 %(w/w), 0.004 %(w/w) to 0.4 %(w/w), 0.004 %(w/w) to 0.3 %(w/w), 0.004 %(w/w) to 0.2 %(w/w), 0.004 %(w/w) to 0.1 %(w/w), 0.004 %(w/w) to 0.08 %(w/w), 0.004 %(w/w) to 0.06 %(w/w), 0.004 %(w/w) to 0.05 %(w/w), 0.004 %(w/w) to 0.04 %(w/w), 0.004 %(w/w) to 0.03 %(w/w), 0.004 %(w/w) to 0.02 %(w/w), 0.004 %(w/w) to 0.01 %(w/w), 0.004 %(w/w) to 0.008 %(w/w), 0.004 %(w/w) to 0.006 %(w/w), 0.004 %(w/w) to 0.005 %(w/w), 0.005 %(w/w) to 2.0 %(w/w), 0.005 %(w/w) to 1.8 %(w/w), 0.005 %(w/w) to 1.6 %(w/w), 0.005 %(w/w) to 1.4 %(w/w), 0.005 %(w/w) to 1.2 %(w/w), 0.005 %(w/w) to 1.0 %(w/w), 0.005 %(w/w) to 0.9 %(w/w), 0.005 %(w/w) to 0.8 %(w/w), 0.005 %(w/w) to 0.7 %(w/w), 0.005 %(w/w) to 0.6 %(w/w), 0.005 %(w/w) to 0.5 %(w/w), 0.005 %(w/w) to 0.4 %(w/w), 0.005 %(w/w) to 0.3 %(w/w), 0.005 %(w/w) to 0.2 %(w/w), 0.005 %(w/w) to 0.1 %(w/w), 0.005 %(w/w) to 0.08 %(w/w), 0.005 %(w/w) to 0.06 %(w/w), 0.005 %(w/w) to 0.05 %(w/w), 0.005 %(w/w) to 0.04 %(w/w), 0.005 %(w/w) to 0.03 %(w/w), 0.005 %(w/w) to 0.02 %(w/w), 0.005 %(w/w) to 0.01 %(w/w), 0.005 %(w/w) to 0.008 %(w/w), 0.005 %(w/w) to 0.006 %(w/w), 0.006 %(w/w) to 2.0 %(w/w), 0.006 %(w/w) to 1.8 %(w/w), 0.006 %(w/w) to 1.6 %(w/w), 0.006 %(w/w) to 1.4 %(w/w), 0.006 %(w/w) to 1.2 %(w/w), 0.006 %(w/w) to 1.0 %(w/w), 0.006 %(w/w) to 0.9 %(w/w), 0.006 %(w/w) to 0.8 %(w/w), 0.006 %(w/w) to 0.7 %(w/w), 0.006 %(w/w) to 0.6 %(w/w), 0.006 %(w/w) to 0.5 %(w/w), 0.006 %(w/w) to 0.4 %(w/w), 0.006 %(w/w) to 0.3 %(w/w), 0.006 %(w/w) to 0.2 %(w/w), 0.006 %(w/w) to 0.1 %(w/w), 0.006 %(w/w) to 0.08 %(w/w), 0.006 %(w/w) to 0.06 %(w/w), 0.006 %(w/w) to 0.05 %(w/w), 0.006 %(w/w) to 0.04 %(w/w), 0.006 %(w/w) to 0.03 %(w/w), 0.006 %(w/w) to 0.02 %(w/w), 0.006 %(w/w) to 0.01 %(w/w), 0.006 %(w/w) to 0.008 %(w/w), 0.01 %(w/w) to 2.0 %(w/w), 0.01 %(w/w) to 1.8 %(w/w), 0.01 %(w/w) to 1.6 %(w/w), 0.01 %(w/w) to 1.4 %(w/w), 0.01 %(w/w) to 1.2 %(w/w), 0.01 %(w/w) to 1.0 %(w/w), 0.01 %(w/w) to 0.9 %(w/w), 0.01 %(w/w) to 0.8 %(w/w), 0.01 %(w/w) to 0.7 %(w/w), 0.01 %(w/w) to 0.6 %(w/w), 0.01 %(w/w) to 0.5 %(w/w), 0.01 %(w/w) to 0.4 %(w/w), 0.01 %(w/w) to 0.3 %(w/w), 0.01 %(w/w) to 0.2 %(w/w), 0.01 %(w/w) to 0.1 %(w/w), 0.01 %(w/w) to 0.08 %(w/w), 0.01 %(w/w) to 0.06 %(w/w), 0.01 %(w/w) to 0.05 %(w/w), 0.01 %(w/w) to 0.04 %(w/w), 0.01 %(w/w) to 0.03 %(w/w), 0.01 %(w/w) to 0.02 %(w/w), 0.03 %(w/w) to 2.0 %(w/w), 0.03 %(w/w) to 1.8 %(w/w), 0.03 %(w/w) to 1.6 %(w/w), 0.03 %(w/w) to 1.4 %(w/w), 0.03 %(w/w) to 1.2 %(w/w), 0.03 %(w/w) to 1.0 %(w/w), 0.03 %(w/w) to 0.9 %(w/w), 0.03 %(w/w) to 0.8 %(w/w), 0.03 %(w/w) to 0.7 %(w/w), 0.03 %(w/w) to 0.6 %(w/w), 0.03 %(w/w) to 0.5 %(w/w), 0.03 %(w/w) to 0.4 %(w/w), 0.03 %(w/w) to 0.3 %(w/w), 0.03 %(w/w) to 0.2 %(w/w), 0.03 %(w/w) to 0.1 %(w/w), 0.03 %(w/w) to 0.08 %(w/w), 0.03 %(w/w) to 0.06 %(w/w), 0.03 %(w/w) to 0.05 %(w/w), 0.03 %(w/w) to 0.04 %(w/w), 0.04 %(w/w) to 2.0 %(w/w), 0.04 %(w/w) to 1.8 %(w/w), 0.04 %(w/w) to 1.6 %(w/w), 0.04 %(w/w) to 1.4 %(w/w), 0.04 %(w/w) to 1.2 %(w/w), 0.04 %(w/w) to 1.0 %(w/w), 0.04 %(w/w) to 0.9 %(w/w), 0.04 %(w/w) to 0.8 %(w/w), 0.04 %(w/w) to 0.7 %(w/w), 0.04 %(w/w) to 0.6 %(w/w), 0.04 %(w/w) to 0.5 %(w/w), 0.04 %(w/w) to 0.4 %(w/w), 0.04 %(w/w) to 0.3 %(w/w), 0.04 %(w/w) to 0.2 %(w/w), 0.04 %(w/w) to 0.1 %(w/w), 0.04 %(w/w) to 0.08 %(w/w), 0.04 %(w/w) to 0.06 %(w/w), 0.04 %(w/w) to 0.05 %(w/w), 0.05 %(w/w) to 2.0 %(w/w), 0.05 %(w/w) to 1.8 %(w/w), 0.05 %(w/w) to 1.6 %(w/w), 0.05 %(w/w) to 1.4 %(w/w), 0.05 %(w/w) to 1.2 %(w/w), 0.05 %(w/w) to 1.0 %(w/w), 0.05 %(w/w) to 0.9 %(w/w), 0.05 %(w/w) to 0.8 %(w/w), 0.05 %(w/w) to 0.7 %(w/w), 0.05 %(w/w) to 0.6 %(w/w), 0.05 %(w/w) to 0.5 %(w/w), 0.05 %(w/w) to 0.4 %(w/w), 0.05 %(w/w) to 0.3 %(w/w), 0.05 %(w/w) to 0.2 %(w/w), 0.05 %(w/w) to 0.1 %(w/w), 0.05 %(w/w) to 0.08 %(w/w), 0.05 %(w/w) to 0.06 %(w/w), 0.1 %(w/w) to 2.0 %(w/w), 0.1 %(w/w) to 1.8 %(w/w), 0.1 %(w/w) to 1.6 %(w/w), 0.1 %(w/w) to 1.4 %(w/w), 0.1 %(w/w) to 1.2 %(w/w), 0.1 %(w/w) to 1.0 %(w/w), 0.1 %(w/w) to 0.9 %(w/w), 0.1 %(w/w) to 0.8 %(w/w), 0.1 %(w/w) to 0.7 %(w/w), 0.1 %(w/w) to 0.6 %(w/w), 0.1 %(w/w) to 0.5 %(w/w), 0.3 %(w/w) to 2.0 %(w/w), 0.3 %(w/w) to 1.8 %(w/w), 0.3 %(w/w) to 1.6 %(w/w), 0.3 %(w/w) to 1.4 %(w/w), 0.3 %(w/w) to 1.2 %(w/w), 0.3 %(w/w) to 1.0 %(w/w), 0.3 %(w/w) to 0.9 %(w/w), 0.3 %(w/w) to 0.8 %(w/w), 0.3 %(w/w) to 0.7 %(w/w), 0.3 %(w/w) to 0.6 %(w/w), 0.3 %(w/w) to 0.5 %(w/w), 0.4 %(w/w) to 2.0 %(w/w), 0.4 %(w/w) to 1.8 %(w/w), 0.4 %(w/w) to 1.6 %(w/w), 0.4 %(w/w) to 1.4 %(w/w), 0.4 %(w/w) to 1.2 %(w/w), 0.4 %(w/w) to 1.0 %(w/w), 0.4 %(w/w) to 0.9 %(w/w), 0.4 %(w/w) to 0.8 %(w/w), 0.4 %(w/w) to 0.7 %(w/w), 0.4 %(w/w) to 0.6 %(w/w), 0.4 %(w/w) to 0.5 %(w/w), 0.5 %(w/w) to 2.0 %(w/w), 0.5 %(w/w) to 1.8 %(w/w), 0.5 %(w/w) to 1.6 %(w/w), 0.5 %(w/w) to 1.4 %(w/w), 0.5 %(w/w) to 1.2 %(w/w), 0.5 %(w/w) to 1.0 %(w/w), 0.5 %(w/w) to 0.9 %(w/w), 0.5 %(w/w) to 0.8 %(w/w), 0.5 %(w/w) to 0.7 %(w/w), or 0.5 %(w/w) to 0.6 %(w/w).

Appropriately mixing the composition with the sample containing the target protein may inhibit or suppress the nonspecific binding of impurities in the sample, and specifically, may inhibit or suppress one or more of: 1) nonspecific binding between impurities in the sample and the target protein, and 2) nonspecific binding between impurities in the sample and the affinity ligand of the affinity chromatography. The nonionic surfactant may effectively remove impurities during the process of purifying the target protein by affinity chromatography, by inhibiting the nonspecific binding of impurities.

The eluate, recovered product, or purified product obtained using the composition may contain a decreased amount of impurities (specifically, host cell proteins) compared to an eluate, recovered product, or purified product obtained without using a composition containing a nonionic surfactant.

The amount of impurities contained in the eluate, recovered product, or purified product obtained using the composition may be decreased by about 5 % or more compared to the amount of impurities contained in an eluate, recovered product, or purified product obtained without using a composition containing a nonionic surfactant. Specifically, the decrease in the amount of impurities may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The eluate, recovered product, or purified product obtained using the composition may exhibit an increased or maintained yield (amount) of the target protein compared to that of the eluate, recovered product, or purified product obtained without using a composition containing a nonionic surfactant, and specifically, may exhibit a yield increased above a certain level or changed (increased/decreased) within a certain allowable range.

The yield (amount) of the target protein contained in the eluate, recovered product, or purified product obtained using the composition may be increased by 5 % or more than the yield (amount) of the target protein of the eluate, recovered product, or purified product obtained without using a composition containing a nonionic surfactant. Specifically, the increase in the yield (amount) of the target protein may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The yield (amount) of the target protein contained in the eluate, recovered product, or purified product obtained using the composition may be increased or decreased by about 10 % or less compared to that of the eluate, recovered product, or purified product obtained without using a composition containing a nonionic surfactant. Specifically, the increase/decrease in the yield (amount) of the target protein may be about 10 % or less, about 8 % or less, about 5 % or less, about 3 % or less, about 2 % or less, about 1 % or less, about 0.5 % or less, about 0.1 % or less, about 0.01 % or less, about 0.01 % to about 10 %, about 0.01 % to about 8 %, about 0.01 % to about 5 %, about 0.01 % to about 3 %, about 0.01 % to about 1 %, about 0.01 % to about 0.1 %, about 0.1 % to about 10 %, about 0.1 % to about 8 %, about 0.1 % to about 5 %, about 0.1 % to about 3 %, about 0.1 % to about 1 %, about 0.5 % to about 10 %, about 0.5 % to about 8 %, about 0.5 % to about 5 %, about 0.5 % to about 3 %, about 0.5 % to about 1 %, about 1 % to about 10 %, about 1 % to about 8 %, about 1 % to about 5 %, or about 1 % to about 3 %.

The eluate, recovered product, or purified product obtained using the composition may contain a decreased amount of impurities (specifically, host cell proteins) than that of the eluate, recovered product, or purified product obtained using a composition (wash buffer) that includes one or more selected from the group consisting of guanidine, guanidine salts, and isopropanol and/or without using a composition containing a nonionic surfactant.

The amount of impurities in the eluate, recovered product, or purified product obtained using the composition may be decreased by about 5 % or more compared to that contained in the eluate, recovered product, or purified product obtained using a composition (wash buffer) that includes one or more selected from the group consisting of guanidine, guanidine salts, and isopropanol and/or without using a composition containing a nonionic surfactant. Specifically, the decrease in the amount of impurities may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The eluate, recovered product, or purified product obtained using the composition may exhibit an increased yield (amount) of the target protein compared to that of the eluate, recovered product, or purified product obtained using a composition (wash buffer) that includes one or more selected from the group consisting of guanidine, guanidine salts, and isopropanol and/or without using a composition containing a nonionic surfactant.

Specifically, the yield (amount) of the target protein in the eluate, recovered product, or purified product obtained using the composition may be increased by about 5 % or more compared to that of the eluate, recovered product, or purified product obtained using a composition (wash buffer) that includes one or more selected from the group consisting of guanidine, guanidine salts, and isopropanol and/or without using a composition containing a nonionic surfactant. Specifically, the increase in the yield (amount) of the target protein may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

### Advantageous Effects of Invention

The composition or method according to an aspect, when used to purify a target protein, can effectively remove HCP while improving the yield of the target protein and may therefore be applicable to manufacturing processes for recombinant protein pharmaceuticals such as antibody therapeutics.

### Brief Description of Drawings

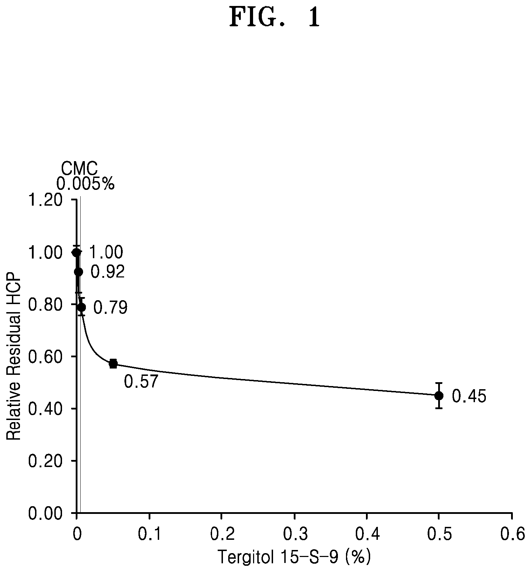

FIG. 1 is a diagram showing results of examining changes in HCP residue in a Protein A chromatography-purified product according to concentration of added Tergitol (0 %(w/w)-0.5 %(w/w)). When the concentration of Tergitol was at least 0.005 %, which is CMC concentration, an increase in HCP removal capacity was observed in proportion to an increase in Tergitol concentration.

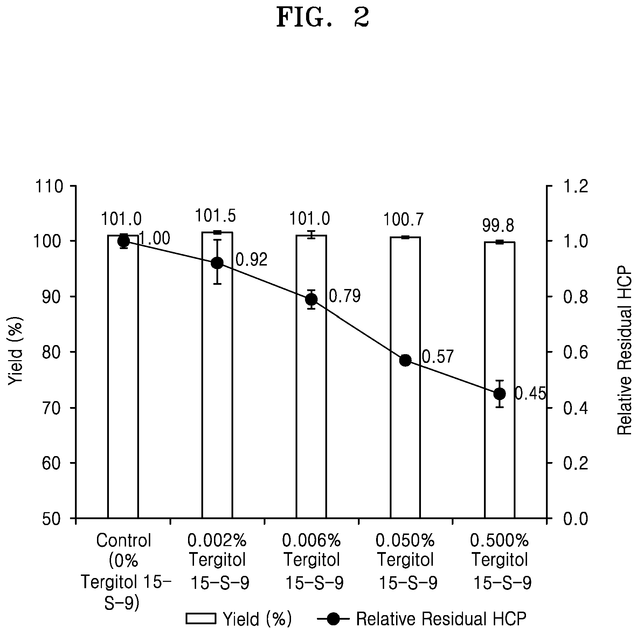

FIG. 2 is a diagram showing results of examining changes in protein yield (process yield) and HCP residue in a Protein A chromatography-purified product according to concentration of added Tergitol (0 %(w/w)-0.5 %(w/w)). Despite the increase in Tergitol concentration, the protein yield remained at a similar level, while the HCP residue showed a tendency to decrease.

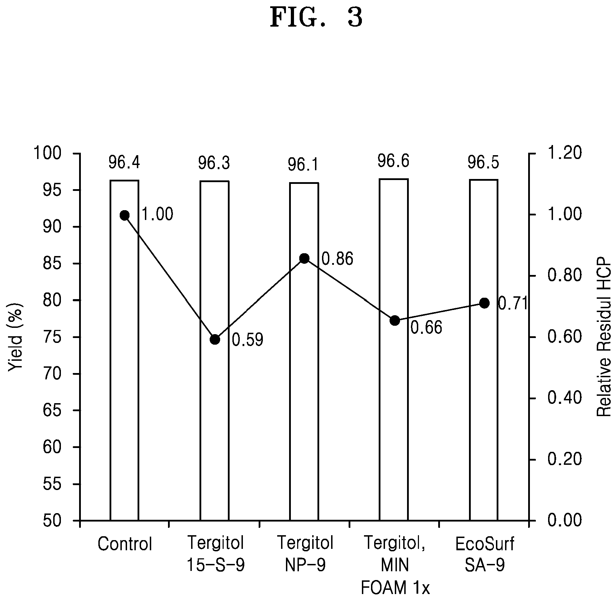

FIG. 3 is a diagram illustrating a comparative analysis of HCP removal capacity depending on the type of nonionic surfactant added (0.5 %(w/w)).

### Best Mode for Carrying out the Invention

### Mode for the Invention

Hereinbelow, the present disclosure will be described in greater detail through experimental examples. However, these embodiments are described for illustrative purposes only and the scope of the present disclosure is not limited to these embodiments.

### Experimental Example 1: Process for Treating HCCF with Nonionic Surfactant

For the purpose of the present disclosure, in technique effectively removing HCP(host cell protein), an impurity present in HCCF (harvest cell culture fluid), the following experiment was conducted to compare and analyze the HCP removal capacity resulting from treatment with a nonionic surfactant. Tergitol was used as an example of the nonionic surfactant.

Specifically, to HCCF obtained from a recombinant protein production process, Tergitol 15-S-9 was added at concentrations in the range of 0 %(w/w) to 0.5 %(w/w) (0.002 %(w/w), 0.006 %(w/w), 0.05 %(w/w), and 0.5 %(w/w)), and the resulting mixture was mixed using a magnetic bar for 30 minutes. Upon completion of the mixing, the mixture was filtered through a 0.22 µm filter. After filtration, the filtrate was incubated at room temperature for at least 0.5 hours.

### Experimental Example 2: Performing Protein A Chromatography

To remove impurities such as HCP (host cell protein) from HCCF and obtain a target protein, an affinity chromatography, specifically Protein A chromatography, was performed as follows.

First, the Protein A column was equilibrated by washing with 5 column volumes (CV) of an equilibration buffer (75 mM sodium phosphate, 100 mM sodium chloride, pH 7.3). Next, HCCF treated with the nonionic surfactant in Experimental Example 1 was loaded onto the Protein A column at an antibody load ratio of 25 g per liter of resin, followed by washing with 3 CV of the equilibration buffer. Next, to remove impurities present in the Protein A column, the column was washed with 3 CV of wash 1 buffer (its detailed composition is listed in Table 1 below).

Then, 3 CV of wash 2 buffer (50 mM Tris, pH 7.2) were applied to remove residual wash 1 buffer in the column prior to antibody recovery. Upon completion of this process, the antibody was recovered from the Protein A column using an elution buffer (300 mM glycine, pH 3.4). The recovered antibody was neutralized to pH 5.5 and then subjected to CHO ELISA analysis (Cygnus CHO HCP ELISA kit, F550-1) for comparison of the results.

### Experimental Example 3: Evaluation of HCP Removal Capacity According to Tergitol Addition and Concentration

In the Protein A chromatography described in Experimental Examples 1 and 2, the yield of the target protein and the HCP concentration (i.e., HCP removal capacity) were measured based on different Tergitol addition concentrations and the composition of the wash 1 buffer. Table 1 below provides the details.

The target protein yield was calculated as the ratio of the total amount of the target protein in the harvest cell culture fluid (HCCF) for each experiment to the total amount of the target protein in the eluate obtained after performing the affinity chromatography.

Specifically, the total amount of the target protein in HCCF was determined by multiplying the volume of HCCF by the HCCF titer, which was measured using Protein G-HPLC. In addition, the total amount of the target protein in the eluate was determined by the eluate volume and the concentration of the target protein in the eluate, and the concentration of the target protein in the eluate was calculated by measuring the absorbance at 280 nm using a UV/Vis spectrophotometer (Because the HCCF contains many impurities besides the antibody, only the antibody is purified via Protein G-HPLC before measuring the titer; while the eluate, having undergone Protein A purification, comprises a highly purified antibody, which allows the concentration to be calculated by measuring the absorbance at 280 nm). Variations depending on the measurement method may occur and lead to protein yields exceeding 100 %.

**[Table 1]**

| No. | Concentration (w/w%) of Turgitol added in TCCF | Wash 1 Buffer Composition | Yield (%) | | HCP (ppm) | | p-value^{a} |
|---|---|---|---|---|---|---|---|
| | N/A | 50 mM Tris, | 101.4 | 101.0 | 1527.5 | 1483 | N/A |
| Control 1 | | 1 M NaCl, pH 7.0 | 101.0 | [SD: 0.3] | 1465.6 | [SD: 39] | |
| | | | 100.8 | | 1456.4 | | |
| Example 1 | 0.002 % | 50 mM Tris,1 M NaCl, pH 7.0 | 101.7 | 101.5 [SD: 0.2] | 1426.1 | 1370 [SD 118] | 0.189 |
| | | | 101.5 | | 1448.9 | | |
| | | | 101.2 | | 1233.7 | | |
| Example 2 | 0.006 % | 50 mM Tris, 1 M NaCl, pH 7.0 | 101.3 | 101.0 [SD: 0.6] | 1214.3 | 1170 [SD: 49] | 0.001 |
| | | | 101.5 | | 1117.6 | | |
| | | | 100.3 | | 1178.7 | | |
| Example 3 | 0.050 % | 50 mM Tris, 1 M NaCl, pH 7.0 | 100.8 | 100.7 [SD: 0.2] | 829.3 | 850 [SD: 20] | 0.000 |
| | | | 100.8 | | 852.6 | | |
| | | | 100.4 | | 868.6 | | |
| Example 4 | 0.500 % | 50 mM Tris, 1 M NaCl, pH 7.0 | 99.9 | 99.8 [SD: 0.2] | 748.2 | 669 [SD: 71] | 0.000 |
| | | | 99.9 | | 610.4 | | |
| | | | 99.6 | | 647.5 | | |
| Control 2 | N/A | 50 mM Tris, 0.8 M Guanidine-HCl, 12 % isopropanol, pH 7.0 | 93.7 | 93.1 [SD: 1.7] | 771.4 | 773 [SD: 11] | 0.000 |
| | | | 91.2 | | 785.3 | | |
| | | | 94.5 | | 763.1 | | |

| | | | | | | | |
|---|---|---|---|---|---|---|---|
| ^{a}2-sample T-test result, comparing to Control 1 condition | | | | | | | |

Specifically, when the Tergitol concentration added to the HCCF was at or below the critical micelle concentration (CMC) of 0.005 %, there was no statistically significant effect compared to that of the Tergitol-free control (Control 1). However, it was found that at concentrations of 0.005 % or higher, the HCP concentration in the Protein A chromatography-purified product markedly decreased as the Tergitol concentration increased (Table 1 and FIG. 1).

Next, examining the process yield of Protein A chromatography and HCP concentration changes according to the Tergitol concentration added to HCCF showed that, as the Tergitol addition concentration increased, the target protein yield remained at a similar level, while the HCP concentration in the purified product showed a tendency to decrease (Table 1 and FIG. 2).

Next, Control 2, which did not add Tergitol in the HCCF but included guanidine in the wash 1 buffer, exhibited some degree of HCP removal, but showed a significantly lower target protein yield compared to Examples 1 to 4 (Table 1).

Accordingly, in a process for purifying or obtaining a target protein from HCCF, the addition of Tergitol at a specific concentration to the HCCF prior to purification effectively removes HCP while simultaneously maintaining or improving target protein yields.

### Experimental Example 4: Evaluation of HCP Removal Capacity According to the Type of Surfactant

To examine the yield (amount) of the target protein and the HCP concentration (i.e., HCP removal capacity) based on the type of nonionic surfactant added to HCCF in the Protein A chromatography described in Experimental Examples 1 and 2, the following experiment was conducted.

Specifically, as the nonionic surfactant added to HCCF, Tergitol 15-S-9, Tergitol NP-9 (CAS No. 127087-87-0), Tergitol MIN Foam 1X (CAS No. 68439-51-0), or EcoSurf SA-9 (CAS No. 68937-66-6) was used, and Protein A chromatography was carried out under the same conditions as in Example 4 of Table 1, except for the type of nonionic surfactant added to the HCCF.

As a result, Tergitol 15-S-9 exhibited a significantly higher HCP removal capacity than the other nonionic surfactants tested (FIG. 3).

Thus, it was found that in a process for purifying or obtaining the target protein from HCCF, adding a secondary alcohol ethoxylate (more specifically, Tergitol 15-S-9) to the HCCF prior to purification allows more effective removal of HCP while maintaining or improving the yield of the target protein, compared to the other types of nonionic surfactants.

While the present disclosure has been described with reference to exemplary embodiments, it will be apparent to those skilled in the art that various changes and modifications may be made without departing from the spirit and scope of the disclosure. Therefore, the embodiments described above should be considered in all respects as illustrative and not restrictive.

## Claims

1. A method of purifying a target protein, the method comprising:
A) mixing a sample comprising a target protein with a composition comprising a nonionic surfactant; and
B) removing impurities from the mixture by using affinity chromatography.

2. The method of claim 1, wherein the nonionic surfactant is at least one selected from the group consisting of Triton X-100, Tergitol 15-S series, and Tergitol NP series.

3. The method of claim 1, wherein the nonionic surfactant comprises at least one selected from the group consisting of Tergitol 15-S-3, Tergitol 15-S-5, Tergitol 15-S-7, Tergitol 15-S-9, Tergitol 15-S-12, Tergitol 15-S-15, Tergitol 15-S-20, Tergitol 15-S-30, and Tergitol 15-S-40.

4. The method of claim 1, wherein the nonionic surfactant comprises alcohol ethoxylate.

5. The method of claim 1, wherein the nonionic surfactant comprises a compound represented by Formula 2: 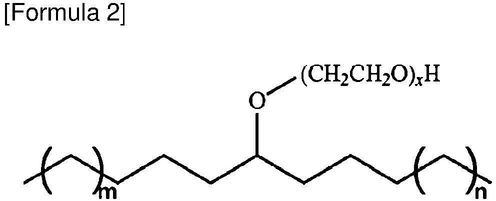
wherein m and n are each an integer greater than 0,
a sum of m and n is an integer of 2 to 6, and
x is an integer of 3 to 40.

6. The method of claim 1, wherein the nonionic surfactant is included at a concentration of 0.003 %(w/w) to 2.0 %(w/w) with respect to a total weight of the mixture.

7. The method of claim 1, wherein the nonionic surfactant inhibits nonspecific binding of the impurities in the sample.

8. The method of claim 1, wherein the sample comprising the target protein is cell culture fluid.

9. The method of claim 1, wherein the affinity chromatography is Protein A chromatography.

10. The method of claim 1, wherein the target protein is a protein comprising a Fc fragment.

11. The method of claim 1, wherein the impurities comprise host cell protein (HCP).

12. The method of claim 1, wherein step B) comprises:
B-1) loading the mixture onto an affinity chromatography column; and
B-2) washing the column with a first wash buffer.

13. The method of claim 12, wherein step B) further comprises B-3) washing the column with a second wash buffer.

14. The method of claim 1, further comprising:
C) recovering the target protein from the column by using an elution buffer.

15. The method of claim 12, wherein the first wash buffer does not include one or more selected from the group consisting of guanidine, guanidine salts, and isopropanol.

16. A method of improving removal of impurities in protein purification by affinity chromatography, the method comprising:
A) mixing a sample comprising a target protein with a composition comprising a nonionic surfactant; and
B) removing impurities from the mixture by using affinity chromatography.

17. A composition for improving removal of impurities in protein purification by affinity chromatography, the composition comprising a nonionic surfactant.
